# EUROPEAN PATENT APPLICATION

(11) **EP 1 752 171 A1**
(43) Date of publication of application: **14.02.2007**
(21) Application number: 05425561.7
(22) Date of filing: 28.07.2005
(51) Int. Cl.: A61M 1/36

(54) **Haemofilters for blood detoxification**

(71) Applicant: Humanitas Mirasole S.p.A., 20089 Rozzano (Milano) (IT); Istituto di Recerche Chimiche e Biochimiche Giuliana Ronzoni, 20133 Milano (IT)
(72) Inventor: Graziani, Giorgio, 20125 Milano (IT); Naggi, Annamaria, 20025 Legnano (IT); Torri, Giangiacomo, 20133 (IT)
(74) Representative: Siniscalco, Fabio

(57) **Abstract**

The present invention relates to haemofilters for the removal of bacterial toxins (lipopolysaccharides) from the blood comprising a solid support onto which cyclodextrins are covalently bound. The solid support may be a fabric or non-woven fabric or a polymeric resin obtained from the cross-linking of cyclodextrins using suitable cross-linking agents, for example epichlorhydrin.

## Description

. The present invention relates to haemofilters for blood detoxification.

**.** Septic syndrome (sepsis) is a serious complication of infection with gram negative bacteria.

. Frequently, said syndrome affects all organs and systems, resulting in multiple organ failure requiring the use of artificial devices (automatic respirators, artificial kidneys and cardiac stimulators). One particularly dreadful and widespread complication of sepsis is septic shock, which can even prove fatal.

**.** Sepsis is triggered by the liberation of exo- and endotoxins from the bacterial outer capsule. Said substances are lipopolysaccharides which, upon entering the circulatory system, trigger a series of reactions involving the humoral and cellular components of the immune system. The situations resulting in the greatest risk of this ailment are: serious trauma, perforations of the abdominal viscera, major abdominal surgery, especially in older diabetic or immune suppressed patients.

**.** Frequently, the body's own reaction to endotoxinaemia can provoke a series of responses involving the organ microcirculatory system (septic cascade).

. This reaction, despite being considered one of the body's defence mechanisms against bacterial invasion, is frequently abnormal, inducing the production of numerous mediators of inflammation (cytokines and other microproteins), responsible for the response's autoamplification process, causing serious damage to organs and systems.

. Treatment with antibiotics and substitution of the damaged organs with artificial devices does not always succeed in stemming the septic cascade.

**.** Hence, the most effective treatment against sepsis is the prevention of the body becoming invaded by bacterial toxins, before the latter can induce the septic cascade.

**.** Lipopolysaccharides (LPS) are characteristic components of the cell membranes of gram negative bacteria; they are not present in gram-positive bacteria.

. They are molecules constituted by a hydrophobic lipid chain which is responsible for the toxic properties, a central hydrophilic polysaccharide chain and a hydrophilic O-polysaccharide side chain, which is specific for each bacterial strain.

. In aqueous solvents, lipopolysaccharides have a tendency to form aggregates of various sizes, particularly micelles having molecular weights of approx. 1,000 KDa. Consequently, they may not be separated from the blood by ultrafiltration, since they do not pass through the membrane pores. Over the years, numerous blood detoxification techniques have been developed, however having the disadvantage of being not very efficient and entailing very high costs, largely due to the materials used. For example, haemofilters comprising antibiotics, capable of detoxifying blood are available. However, such filters have very high costs.

. The aforecited problems are resolved by a haemofilter as defined in the enclosed claims.

. In a first aspect, the present invention relates to a haemofilter comprising a solid support to which cyclodextrins or cyclodextrin derivatives are covalently bound.

**.** The solid supports used in the invention may be polymeric resins obtained through a cross linking reaction between the cyclodextrins and suitable cross-linking agents, for example epichlorhydrin, isocyanates, polyamines, acrylates; or fibre or fabric supports, for example cellulose, or non-woven fabric, for example polypropylene, to which the cyclodextrins are bound by means of chemical or physical treatments such as the use of electron beams. From the above options, the preferred supports are those made of natural cellulose.

**.** Cyclodextrins (CDs), also known as cycloamylose, cycloglucans, cyclomaltosides, are cyclic oligosaccharides constituted by the joining together, through α(1,4) bonds, of glucose units in numbers varying between 6 and 12. The term CD is preceded by a Greek letter indicating the number of glucose units present in the ring (α corresponds to 6 units, β corresponds to 7 units, etc.).

**.** Cyclodextrins are the enzymatic breakdown product of starch, by the enzyme glycosyl transferase (CGTase) produced by various bacteria (*e.g.: Bacillus Macerans, Klebsiella Pneumonite, Bacillus Stearothermophilus, Bacillus Megaterium* etc.).

**.** The enzyme CGTase cleaves the helical structure of the starch molecule, while at the same time forming α(1,4) bonds between the glucose molecules, leading to the formation of cyclic oligoglucosides.

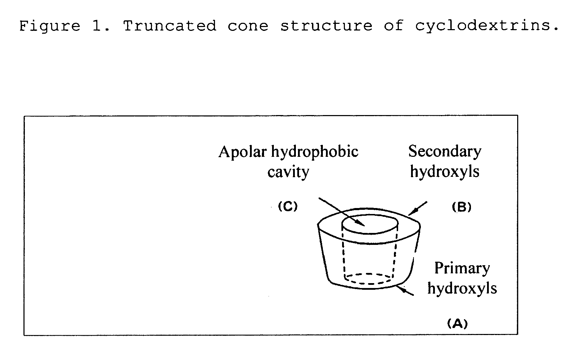

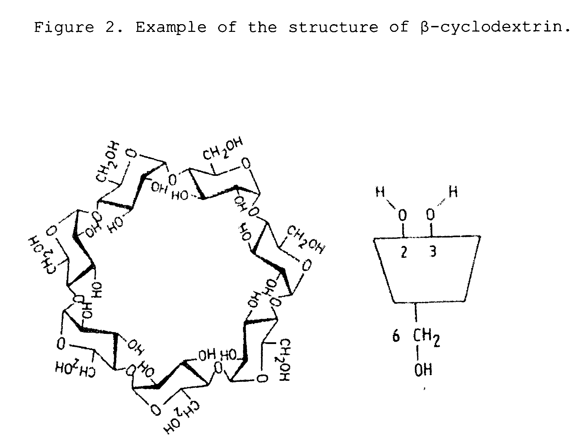

. Cyclodextrins, as shown in figures 1 and 2, have a truncated conical structure with the hydroxyl groups facing outwards, and the carbon and hydrogen atoms and the hetero-oxide bonds towards the inside of the structure. Furthermore, they have the primary hydroxyl groups positioned in the truncated cone area with the smaller diameter, and the secondary groups in the area with the larger diameter.

. This structure confers the CDs with particular properties: the high electron density, caused by the glycosidic oxygens, makes the cavity of the molecule apolar and hydrophobic and confers the exterior with a hydrophilic nature.

. Hence the principle on which the invention is based is that of binding water soluble cyclodextrins to a solid support. Blood, in particular blood plasma, is then made to pass over said support. The hydrophobic cavity inside the cyclodextrins retains the bacterial endotoxins, thus allowing the detoxification of the blood, which is then returned to clean circulation.

**.** Detoxification is possible since the endotoxins or lipopolysaccharides are hydrophobic molecules and thus form a stable and reversible complex, known as an inclusion complex, with the hydrophobic cavity inside the cyclodextrins.

. In the majority of cases, the ratio between CDs and the bound molecule is one to one, and no covalent bonds are involved, however an associative-dissociative type equilibrium is established, mainly due to Van der Waals interactions and hydrogen bonding between the included molecule and the hydroxyl groups on the CD.

**.** For the purposes of the invention, the preferred cyclodextrins are alpha, beta and gamma cyclodextrins and derivatives thereof, such as hydroxypropyls, sulphates and ethylsulphonates, preferably alpha cyclodextrin.

. Synthesis of the solid polymeric support occurs by making the cyclodextrins react with suitable cross-linking agents. Said cross-linking agents are advantageously bifunctional molecules having a functional group capable of reacting with the primary and secondary hydroxy groups of the cyclodextrins (for example epoxides and halides) at either end.

**.** Examples of cross-linking agents include epichlorhydrin, isocyanates, polyamines, acrylates, preferably, it is epichlorohydrin. The method for the synthesis is known in the literature, for example in the following article: E. Renard, G. Barnathan, A. Deratani and B. Sebille, "Characterization and structure of cyclodextrin-epichlorohydrin polymers-effects of synthesis parameters, (1996), Proceeding of the Eighth International Symposium on Cyclodextrins.

. The polymers synthesised are in the form of granules, which are insoluble in water and also mostly insoluble in organic solvents (alcohol, chloroform, acetone, DMF, DMSO etc.).

. For the purposes of the invention, the polymers preferentially used are polymers obtained from the cross-linking of cyclodextrins with epichlorohydrin, having a cyclodextrin content of 200 to 900 µmol/g, preferably 600-800 µmol/g.

. Grafting of the cyclodextrins onto a yarn or fabric, for example cellulose, or onto a polymeric non-woven fabric, for example cellulose acetate, polypropylene, polyethylene, polyester, takes place by interposing a linker monomer between the substrate and the cyclodextrins.

. There are several monomers which can be used in the grafting reaction, for example glycidyl methacrylate (GMA), acrylic acid, N-vinylpyrrolidone, acrylamide and vinylacetate. Preferably, the monomer used is glycidyl methacrylate.

**.** GMA monomers are of particular interest thanks to the presence of an extremely reactive functional group, *viz*. epoxide.

. The method used for grafting the monomer onto the substrate is described in the literature, for example in the following articles: P. Le Thuaut, G. Crini, M. Morcellet, A. Naggi, U. Maschke, C. Vecchi, X. Coqueret, G. Torri and B. Martel, J. Appl. Polym. Sci., (1997); P. Le Thuaut, Ph.D. thesis in organic and macromolecular chemistry, Lille University of Science and Technology, Chemistry and Macromolecular Laboratory, "Fonctionnalisation de supports textiles pour l'élaboration de filtres adsorbeurs de polluants organiques", (2000). Typically, synthesis involves the following steps:
- Activation of the solid substrate by means of chemical or physical treatment, for example with a beam of electrons etc. The preferred technique is irradiation using an electron beam;
- radical-based monomer grafting reaction leading to both the derivatisation of the substrate and the polymerisation of the monomer generating spacers of various lengths.

. Once the spacer is grafted onto the substrate the latter is functionalised using cyclodextrin. Functionalisation methods, involving CD, for the materials used in the present invention are described in the literature, for example in the following articles: K. Poulakis, H.J. Buschmann and E. Schollmeyer, Patent DE 40 35378 A1, (1992); H. Reuscher and R. Hirsenkorn, EP 0 697 415 A1, (1995); H. Reuscher and R. Hirsenkorn, Patent DE 19 520 967, (1995); P. Le Thuaut, G. Crini, M. Morcellet, A. Naggi, U. Maschke, C. Vecchi, X. Coqueret, G. Torri and B. Martel, J. Appl. Polym. Sci., (1997).

. In a second aspect, the present invention relates to a method for blood detoxification comprising the following steps:
a) Removing blood from a patient at risk of sepsis;
b) Separating the plasma from the remaining blood fractions by inserting a plasma filter made of polysulphone or derivatives thereof, for example polyethersulphone, in the arterial line of the extracorporeal circuit;
c) Filtering the plasma through the haemofilter according to the invention;
d) Combining the blood plasma with the previously separated blood fraction.

**.** Once step d) has been completed, the blood, thus detoxified, may be immediately retransferred to the patient.

**.** In a third aspect, the invention relates to the use of a haemofilter and the blood detoxification method described above in cases of blood poisoning, caused by the erroneous consumption of certain classes of drugs, for example barbiturates, or other poisonous substances. These substances, such as lipopolysaccharides, are complexed by the immobilised cyclodextrins and hence removed from the blood.

. EXPERIMENTAL DATA

**.** Synthesis of the polymer support material.

. Cyclodextrins may be polymerised by making one of the hydroxyl groups react with the bifunctional molecule epichlorhydrin. In alkali environments, epichlorhydrin may react with CD (cross-linking reaction) and/or with itself (homopolymerisation), as shown in reaction scheme 1, leading to the synthesis of the polymer shown in scheme 2.

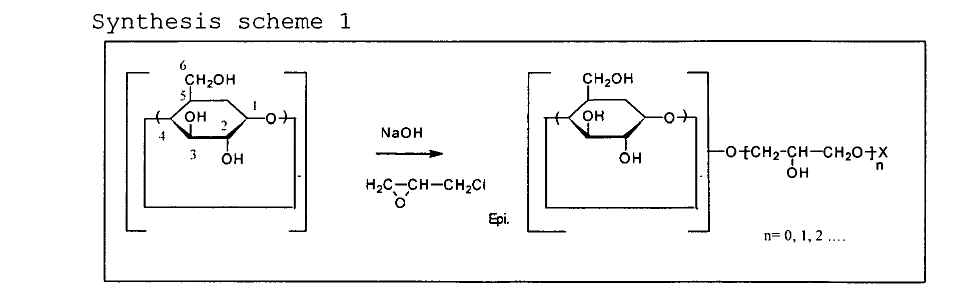

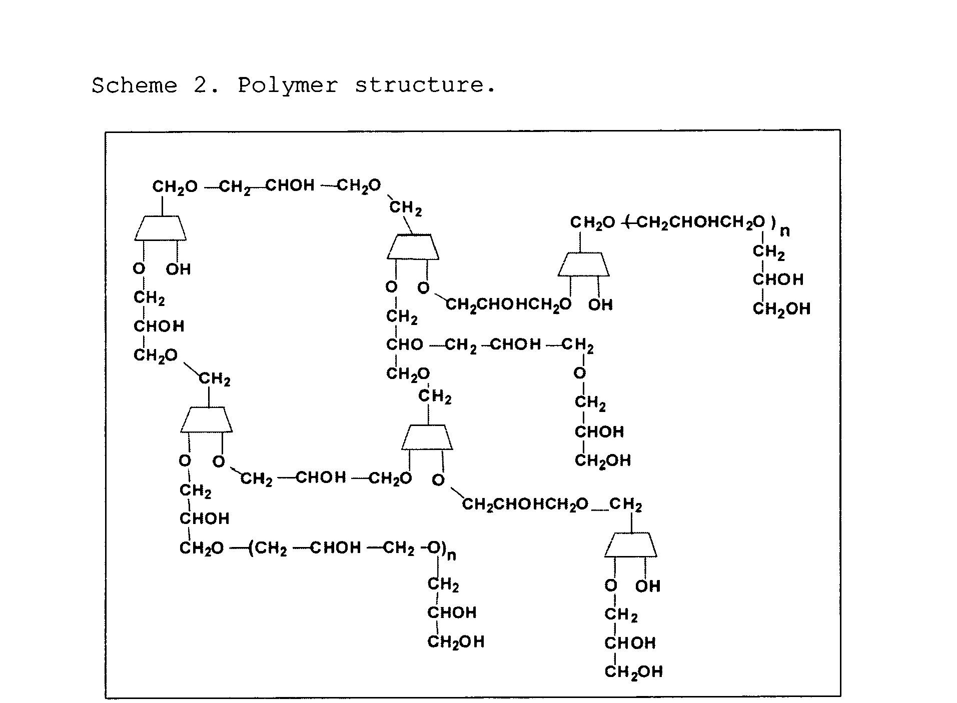

. In a thermostatically controlled reactor with mechanical stirring, aqueous solutions of various percentage concentrations of cyclodextrin (Wacker) and NaOH (Carlo Erba) are mixed. After stirring for one hour, the desired quantity of epichlorhydrin (Fluka) is added slowly, dropwise and the formation of a highly viscous whitish paste is immediately observed, which is then kept stirring vigorously for the various lengths of time described in table 1. Acetone (Acros) is then added, and the product recovered by filtration through a Gooch crucible. Excess un-reacted epichlorhydrin and cyclodextrin are eliminated by washing the polymer with hot water and ethanol (Girelli) in Soxhlet extractor. Finally, the recovered product is dried by lyophilisation.

**.** In order to determine the quantity of CD present in the synthetic products, glucose, obtained by the total hydrolysis of the polymers, has been quantified by colourimetric dosage using phenol.

**.** For this purpose, 20 mg of polymer are suspended in 5 ml of 1M trifluoroacetic acid (Fluka), the suspension is heated with vigorous stirring for eight hours at 120°C. The solution is subsequently evaporated in order to eliminate the trifluoroacetic acid. The product is then taken up in 10 ml of distilled water. The glucose content is determined by colourimetric dosage using phenol and sulphuric acid. The method requires a calibration curve: for this purpose, solutions of varying concentrations of glucose (Fluka) as shown in the table, are prepared, starting from a 1% by weight stock glucose solution (10g/l).

| *Volume of glucose soln. (µl)* | *Volume of water (µl)* |
|---|---|
| 0 | 500 |
| 20 | 480 |
| 30 | 470 |
| 40 | 460 |

. The same procedure is repeated for the hydrolysed polymer solution. Indeed, various volumes are taken from the 10 ml of neutral solution and diluted with various amounts of water as reported in the table:

| *Volume of glucose soln. to be measured (µl)* | *Volume of water (µl)* |
|---|---|
| 20 | 480 |
| 50 | 450 |
| 100 | 400 |

**.** To the above solutions, in test tubes, are added 0.5 ml of a 5% by weight aqueous solution of phenol (Carlo Erba). After 10 minutes have elapsed, 2.5ml of 98% H₂SO₄ (Carlo Erba) are added while stirring vigorously. After 10 minutes following the addition, the absorbances are read at a wavelength of 480 nm. By using Beer-Lambert's law it is possible to construct a calibration curve and hence determine the glucose content in the hydrolysed polymer solution. This way the cyclodextrin content, expressed as µmoles or µg per g of material, is obtained.

. Evaluation of the amount of cyclodextrin present in the various synthesised polymers has allowed the division of the materials into two groups: low (~300 µmol/g) and high (~600/800 µmol/g) CD content.

**.** Table 1 reports the various experimental conditions and the CD values obtained.

**Table 1: Description of the polymers obtained**

| | | | | | | | |
|---|---|---|---|---|---|---|---|
| Sample | CD (g.) | Epi./CD (molar ratio) | *V NaOH* (23%w/w) | Time (h) | T (°C) | % CD | |
| | | | | | | (w/w) | µmol/g |
| Gel II | 3 | 20/l | 80ml | 24 | 80 | 20 | 176 |
| 57% | 3 | 20/l | 4ml | 5 | 50 | 57 | 502 |
| 60% | 3 | 20/l | 4ml | 2 | 50 | 60 | 527 |
| 70% | 3 | 20/l | 3ml | 5 | 50 | 70 | 617 |
| 80% | 3 | 60/l | 3ml | 24 | 50 | 80 | 705 |
| Gel βCD* | 350 | 20/l | 500ml | 24 | 50 | 84 | 740 |
| Gel γCD* | 350 | 15/l | 150ml (50% w/w) | 4 | 50 | 73 | 562 |
| Gel α CD* | 350 | 15/l | 150ml (50% w/w) | 2 | 50 | 31 | 319 |

| | | | | | | | |
|---|---|---|---|---|---|---|---|
| * polymerisation carried out using equipment simulating a pilot plant | | | | | | | |

**.** The material, denoted in the table as Gel II, has been synthesised using the known method from the literature (E. Renard, G. Barnathan, A. Deratani and B. Sebille, "Characterization and structure of cyclodextrin-epichlorohydrin polymers-effects of synthesis parameters, (1996), Proceeding of the Eighth International Symposium on Cyclodextrins).

. This material has been used as a reference point for both the characterisation and complexing capacity evaluation studies.

. Thanks to the modifications to the method in the literature, working with small volumes of sodium solutions, polymers with notably higher CD contents compared to the sample reference, Gel II, are attained.

**.** From analysis of the experimental conditions employed for synthesis, it may be concluded that the fundamental parameter for achieving high cyclodextrin content materials is the amount of solvent present in the reaction mixture.

**.** Synthesis of the solid supports on fabric or non-woven fabric.
Products are prepared in accordance with the method described in "Grafting of cyclodextrins onto polypropylene on-woven fabrics for the manufacture of reactive filters. II Characterisation" B. Martel, P. Le Thuaut, G. Crini, M. Morcellet, A. Naggi, U. Maschke, S. Bertini, C. Vecchi, X. Coqueret, and G. Torri J. Appl. Polym. Sci., 78: 2166-2173, (2000) in accordance with the scheme described in figure 3. The non-woven fabric is activated physically by using between 5 to 30 Mrad from a device providing an elementary activation dose by means of a 50 KGy (5Mrad) electron beam, with the irradiation steps being repeated in order to attain higher doses. The glycidylmethacrylate grafting reaction occurs by immersing the activated fabric in a water bath containing the monomer at concentrations ranging from 1 to 70% and heating it to 70°C in an inert atmosphere for periods of time varying between 20 and 200 minutes. Quantification of the bound GMA is assessed from the fabric's weight increase following prolonged washing and drying cycles. Derivatisation with cyclodextrin occurs by immersing the functionalised fabric in a solution of cyclodextrin in DMF/H₂O and heating it to 80°C for periods of time comprised between 8 and 24 hours. The quantity of cyclodextrin introduced is assessed from the fabric's weight gain following prolonged washing and drying cycles. Reaction scheme 3

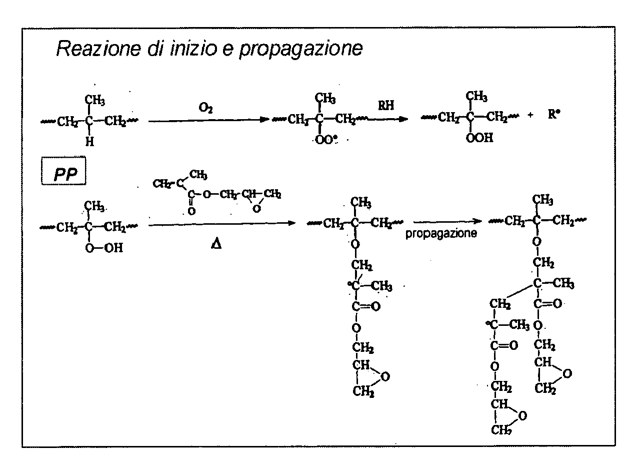

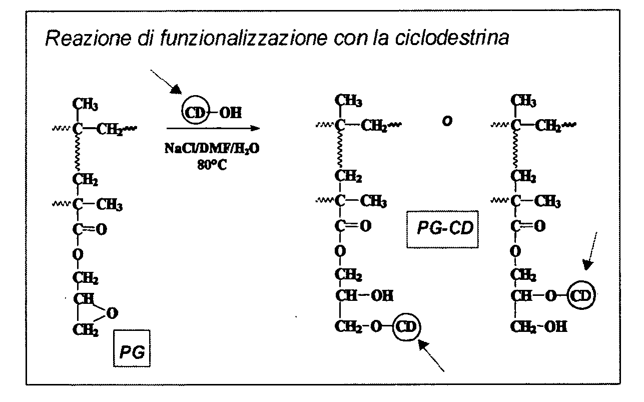

### ADVANTAGES

**.** The haemofilter according to the invention uses a material, cyclodextrin, which is easily obtained and hence very economical. This allows significant reductions in the costs of the individual haemofilters, highly advantageous to the health of those patients affected by sepsis.

**.** Furthermore, the blood detoxification efficiency is much greater in comparison to the filters currently available.

. With the haemofilters of the invention it is sufficient to perform a single plasma filtration cycle in order to obtain the complete elimination of all endotoxins, in comparison to the detoxification systems of the known art requiring repeated applications in order to achieve the same results.

## Claims

1. A haemofilter comprising a solid support, to which cyclodextrins or cyclodextrin derivatives are covalently bound.

2. The haemofilter according to claim 1 wherein said solid support is a polymeric resin.

3. The haemofilter according to claim 2 wherein said polymeric resin is cyclodextrin, cross-linked using a cross-linking agent selected from: epichlorhydrin, isocyanates, polyamines, acrylates.

4. The haemofilter according to claims 2 or 3 wherein said polymeric resin is cyclodextrin, cross-linked using epichlorhydrin, with a cyclodextrin content of 200 to 900 µmol/g.

5. The haemofilter according to claim 4 wherein said cyclodextrin content is of 600 to 800 µmol/g.

6. The haemofilter according to claim 1 wherein said solid support is a yarn or fabric or non-woven fabric.

7. The haemofilter according to claim 6 wherein said fabric is cellulose and said non-woven fabric is polypropylene, polyethylene, polyester or cellulose acetate.

8. The haemofilter according to claims 6 or 7 wherein said non-woven fabric is polypropylene.

9. The haemofilter according to any of the claims 1 to 8 wherein said cyclodextrin is bound to yarn or to fabric or to non-woven fabric through a linker monomer.

10. The haemofilter according to claim 8 wherein said linker monomer is selected from: glycidyl methacrylate (GMA), acrylic acid, N-vinylpyrrolidone, acrylamide or vinylacetate.

11. The haemofilter according to claim 10 wherein said linker monomer is glycidyl methacrylate (GMA).

12. A method for the detoxification of blood comprising the following steps:
a) Removing blood from a patient at risk of sepsis;
b) Separating the plasma from the remaining blood fractions;
c) Filtering the plasma using the haemofilter according to any of the claims 1 to 10;
d) Combining the blood plasma with the previously separated blood fraction.

13. The method according to claim 12 comprising, following step d), a step involving re-transferring the purified blood to the patient.

14. The method according to claims 12 or 13 wherein bacterial endotoxins and exotoxins are removed from the blood.

15. The method according to claim 14 wherein said bacterial endotoxins and exotoxins are lipopolysaccharides from gram-negative bacteria.

16. The method according to any of the claims 12 to 15 for the prevention of septic syndrome (sepsis).

17. The method according to any of the claims 12 to 16 for the removal of drugs and/or poisonous substances, such as barbiturates, from the blood.

18. Use of the haemofilter according to any of the claims 1 to 11 for blood detoxification.

19. The use according to claim 18, in cases of intoxication caused by the erroneous consumption of poisonous substances and/or drugs.
